# EUROPEAN PATENT SPECIFICATION

(11) **EP 1 609 874 B1**
(45) Date of publication and mention of the grant of the patent: **05.11.2008**
(21) Application number: 05011112.9
(22) Date of filing: 23.05.2005
(51) Int. Cl.: C12Q 1/68, C12Q 1/70

(54) **System for searching for and identifying pathogenic agents**
System zur Suche und Identifizierung von pathogenen Agenzien
Système pour la recherche et identification d' agents pathogéniques.

(30) Priority: 21.05.2004 IT MO20040126
(43) Date of publication of application: 28.12.2005
(60) Divisional application: 07103358.3
(73) Proprietor: BCS BIOTECH S.p.A., 09131 Cagliari (IT)
(72) Inventor: Perseu, Sinibaldo, Quartu Sant'Elena (CA) (IT); De Montis, Antonella, Cagliari (IT); Lautero, Cassandra, Cagliari (IT); Manca, Ilaria, Pirri (CA) (IT)
(74) Representative: Gervasi, Gemma

(56) References cited:
- WO-A-01/68915
- WO-A-01/85994
- WO-A-03/014397
- WO-A-03/027323
- WO-A-03/087829
- US-A- 4 889 818
- US-A- 5 565 320
- PATENT ABSTRACTS OF JAPAN vol. 2003, no. 12, 5 December 2003 (2003-12-05) & JP 2003 310257 A (YAMAGUCHI TECHNOLOGY LICENSING ORGANIZATION LTD), 5 November 2003 (2003-11-05)
- DELRIO-LAFRENIERE S A ET AL: "LOW-DENSITY ADDRESSABLE ARRAY FOR THE DETECTION AND TYPING OF THE HUMAN PAPILLOMAVIRUS" DIAGNOSTIC MICROBIOLOGY AND INFECTIOUS DISEASES, ELSEVIER SCIENCE PUBLISHING CO., AMSTERDAM, NL, vol. 48, no. 1, January 2004 (2004-01), pages 23-31, XP001197394 ISSN: 0732-8893
- PARK TAE CHUL ET AL: "Human papillomavirus genotyping by the DNA chip in the cervical neoplasia." DNA AND CELL BIOLOGY, vol. 23, no. 2, February 2004 (2004-02), pages 119-125, XP002357729 ISSN: 1044-5498
- HWANG TAE SOOK ET AL: "Detection and typing of HPV genotypes in various cervical lesions by HPV oligonucleotide microarray." GYNECOLOGIC ONCOLOGY, vol. 90, no. 1, July 2003 (2003-07), pages 51-56, XP002357730 ISSN: 0090-8258
- KIM CHAN JOO ET AL: "HPV oligonucleotide microarray-based detection of HPV genotypes in cervical neoplastic lesions." GYNECOLOGIC ONCOLOGY, vol. 89, no. 2, May 2003 (2003-05), pages 210-217, XP002357731 ISSN: 0090-8258
- WILSON W J ET AL: "Sequence-specific identification of 18 pathogenic microorganisms using microarray technology" MOLECULAR AND CELLULAR PROBES, vol. 16, no. 2, April 2002 (2002-04), pages 119-127, XP001152529 ISSN: 0890-8508

## Description

The invention relates to a system for simultaneously searching for and identifying various pathogenic agents that are responsible for common and/or serious human pathologies, which may be found in biological samples of human origin such as pharyngeal swabs, urogenital swabs, bronchial aspirates, blood, biopsies and faeces.

The system can furthermore be used, by means of minimal modifications, for rapidly and simultaneously identifying polymorphisms and/or genetic alterations predisposing to certain neoplastic and/or genetic pathologies, e.g. familiar coagulation disturbances.

In the microbiological laboratories, identification of a pathogenic agent in a biological sample is usually performed in direct manner, that is through cultural examination of the sample.

A drawback of the aforementioned procedure consists of the fact that the latter, in addition to constituting a potential biological risk for the assigned operators, is often laborious, and requires at least 48-hour incubation of the sample. This increase in work time causes a consequent increase in analysis costs.

Furthermore, prolonged time taken to conduct the analysis leads to a significant delay in the delivery by a laboratory of the respective analytical result. This is potentially damaging, for example for a patient waiting to receive suitable treatment when the latter has to be based on the aforementioned analytical result.

Another drawback consists of the fact that many human and animal pathogenic agents are difficult or impossible to propagate in a culture, which makes it significantly difficult to apply the known analytical procedures.

A further drawback consists of the fact that often pathogenic agents that are able to produce similar clinical symptoms have very different culture features, which make it impossible to conduct a simultaneous search for these pathogenic agents by conducting a single analytical procedure.

In order to overcome the drawbacks of the prior art, microbiological analysis methods have been proposed that are based on molecular biology. The latter do not require the culture growth of the microorganism to obtain the identification thereof, as they are not based on the recognition of particular biochemical and/or serological features of the microorganism but on the identification of specific target gene sequences that are present in the genome of the microorganism. In order to obtain this identification, it is thus necessary to know, at least partially, the genome of the searched microorganism, and this is made possible by the fact that various databases exist from which the partial or complete sequences of most pathogenic agents are obtainable.

Identification methods have thus been devised that enable specific target sequences to be identified, if the latter are present in a sample, enable these sequences to be amplified by PCR technique and enable a final result (presence/absence) to be provided by analysing the amplified product by means of electrophoresis on agarose gel.

The International patent Application WO 03/027323 describes a genotyping kit for diagnosis of human Papilloma virus Infection based on specific sequences of several HPV subtypes, preferably complementary to the L1 region of HPV.

The patent Application WO 01/85994 describes a method for identifying HPV viruses with primers representative of the E6 and E7 region of middle, low and high-risk HPV subtypes.

Nevertheless, a drawback associated with the known methods based on PCR consists of the fact that the electrophoretic analysis alone does not prevent these methods, despite their considerable sensitivity, from providing misleading results, i.e. false positives or false negatives.

An object of the invention is to improve known microbiological analysis methods.

Another object is to provide an analytical method that enables pathogenic microorganisms that are taxonomically different from one another to be simultaneously searched for and identified in biological samples.

Another further object is to improve analytical screening methods in the microbiological field.

A further other object is to provide a screening system for identifying pathogenic agents in biological samples.

In a first aspect of the invention, a chip is provided for searching for and identifying pathogenic agents, comprising a solid support and oligonucleotidic probes adhering to preset sites of said solid support, said oligonucleotidic probes being specific for genic sequences of said pathogenic agents. Methods for searching for and identifying pathogenic agents in a sample, comprise:
- extracting genomic nucleic acids from said sample;
- simultaneously amplifying a plurality of genic sequences comprised in said genomic nucleic acids, so as to produce amplified genic sequences;
- obtaining hybridization between said amplified genic sequences and specific probes bound to preset sites of miniaturized solid support means;
- highlighting said hybridization by means of a colorimetric reaction such as to give rise to a specific pattern on said miniaturized solid support means.

In an embodiment, the miniaturized solid support means comprises a chip.

Accordingly, a miniaturized solid support is provided for searching for and identifying pathogenic agents in a sample, comprising gene amplifying means for obtaining an amplification of specific genic sequences contained in nucleic acids of said pathogenic agents, and probes arranged to be hybridised with said genic sequences after said amplification, with highlighting means arranged to highlight said hybridisation.

In such a way the screening system enables various viral pathogenic agents in a same sample to be searched for and to be rapidly identified.

The highlighting means produces a colorimetric reaction that highlights the completed hybridisation of specific sequences with respective probes bound to the surface of the miniaturized solid support means, which can be the well of a glass poly-chip or the well of a microplate. This result can be read with the naked eye, if chips having an area of >1 cm² (poly-chip) are used or by means of a scanner if chips having an area of <1 cm² (microplate) are used.

The possibility of searching for and identifying a plurality of taxonomically distinct microorganisms in a same sample and during a single analysis arises from the fact that specific probes for reciprocally different pathogenic agents can be fixed on the surface of a single chip, in numerous and preset positions (addresses) so as to form specific reciprocally different patterns. In this way, it is possible to simultaneously test for the presence of a vast range of specific sequences that are reciprocally different in a same amplified product and on a same chip.

Unlike what occurs in the methods that provide for the use of electrophoresis to analyse the PCR-amplified product, the method provided by the invention enables a substantially unequivocal result to be obtained and which is thus not interpretable in an incorrect manner. In fact, the amplified product obtained by PCR is hybridised with specific probes directed to "internal" sequences in relation to those used for amplification, which, in addition to increasing the sensitivity of the analytical method, enables the specificity of the latter to be significantly improved.

In a fourth aspect of the invention, a method is provided for interpreting an analytical result, comprising:
- carrying out a scan through optic reader means of a surface of a miniaturized solid support means, said surface comprising sites at which hybridisation between amplified genic sequences and specific probes has been induced, so as to obtain an image of said sites;
- comparing said image with a reference image, so as to verify whether and in which of said sites said hybridisation has been obtained.

In an embodiment, the interpreting method is performed by a programme that can be run on a computer system.

Owing to this aspect, it is possible to interpret precisely the results of an analysis conducted using chips having dimensions of <1 cm².

In another embodiment, the interpreting method provides for acquiring data that relate to the sample and are contained in barcode means using further optic reader means, and automatically associating such data with the analytical results obtained.

In this way, it is possible to manage jointly the data expressing the analytical results with other data relating, for example, to the type and/or the origin of an analysed sample, thus simplifying the management of the analytical results.

The invention will be better understood and implemented with reference to the attached drawings, which illustrate some exemplifying but non-limitative embodiments thereof, in which:
Figure 1 is a schematic exploded side view of a container for miniaturized solid supports;
Figure 2 is a schematic plan view showing an arrangement of specific probes on a miniaturized solid support;
Figures 3 to 32 are schematic plan views showing various patterns that are obtainable on the basis of the arrangement of specific probes in Figure 2;
Figure 33 is an exploded perspective view of optic reader means;
Figure 34 is another exploded perspective view of the optic reader means in Figure 33;
Figure 35 is a further exploded perspective view of the optic reader means in Figure 33;
Figure 36 is a diagram showing an arrangement of optic means comprised in the optic reader means in Figure 33;
Figure 37 is a schematic plan view of a microplate in which a plurality of miniaturized solid supports are obtained;
Figure 38 is a flow diagram showing the operation of the optic reader means in Figure 33;
Figure 39 is a flow diagram showing an operating arrangement comprising the optic reader means in Figure 33, further optic reader means and computer means;
Figure 40 is a flow diagram showing an operating phase of the operating arrangement in Figure 39;
Figure 41 is a flow diagram showing a further operating phase of the operating arrangement in Figure 39.

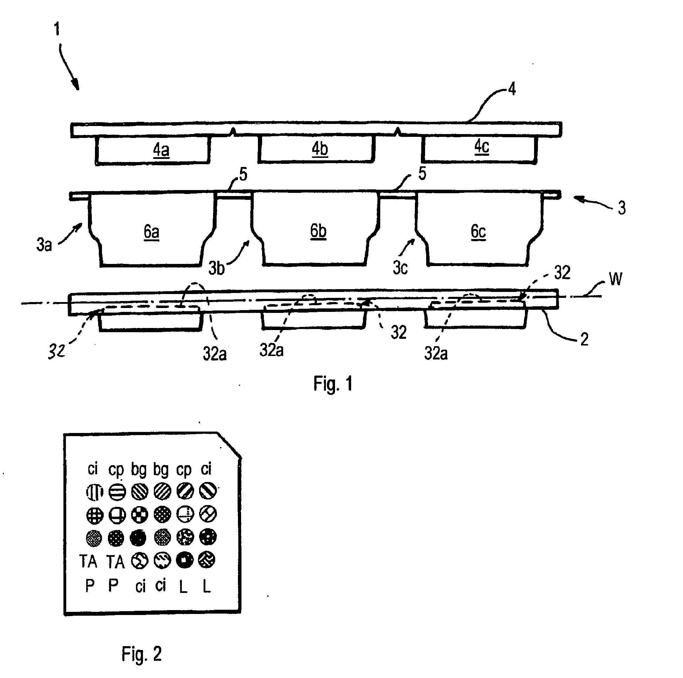

With reference to Figure 1, a container 1 is provided that is arranged to contain simultaneously three miniaturized solid supports, comprising a base 2, a seal 3 and a lid portion 4. In the rectangular base 2, three seats are obtained (that are not shown) that are reciprocally aligned according to and are arranged parallel to a longitudinal axis W of the base 2. In each seat a chip 32 is located (shown by a broken line) having an area of > 1 cm², arranged in such a way that a face 32a thereof faces in the direction opposite the base 2. The seal 3 comprises three hollow elements 3a-3c, each of which is approximately parallelepiped-shaped and is delimited by a respective side wall 6a-6c. The hollow elements 3a-3c are aligned parallel to the longitudinal axis of the base 2 and are connected together by bridge portions 5. The seal 3 can be fitted to the base 2 in such a way as to make each hollow element 3a-3c match a respective seat. In this way, each seat, and thus each chip 32 that is contained therein, is positioned on the bottom of a well defined by the side wall 6a-6c of the corresponding hollow element 3a-3c. The lid portion 4, which is rectangle-shaped, comprises three closing elements 4a-4b, aligned reciprocally and arranged parallel to the longitudinal axis W. Each closing element 4a-4b, which is quadrilateral-shaped, is positioned along the lid portion 4 in such a way as to be able to be inserted into a corresponding well, such as to close the latter when the lid portion 4 is applied to a side of the seal 3 opposite the base 2.

The container 1, in addition to the embodiment disclosed above comprising three chips 32 (poly-chips), can be made in an embodiment (not shown) that is arranged to house a single chip.

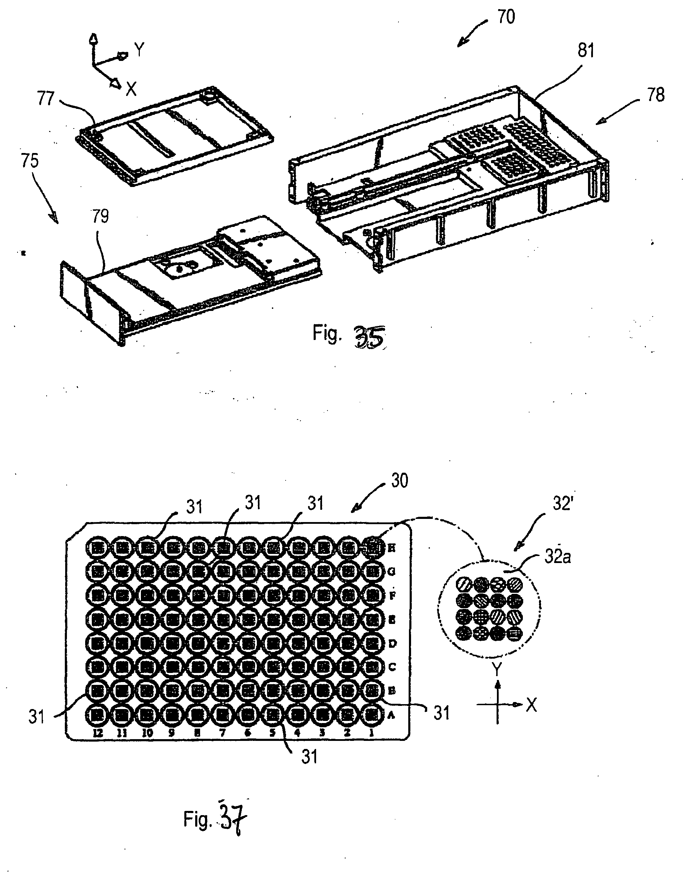

With reference to Figure 37, a microplate 30 is provided, having a rectangular plan shape, in which a plurality of wells 31 are obtained. On the bottom of each well 31 a chip 32' is obtained having an area of < 1 cm², the face 32a of which well faces a direction opposite the bottom of the well 31.

In an embodiment, the microplate 30 is obtained from a 96-well plate for microtitration (MTP) of the known type.

A variable and preset number of specific probes, comprised between a minimum of 2 and a maximum of 10000, is fixed to the face 32a of each chip 32, 32'. The probes may be spotted in preset positions or addresses of the solid support through one of the many commercially available apparatuses, for example the "SciFlex arrayer S11" (Scienion AG, Germany). This apparatus uses non-touch piezoelectric spotting technology and is able to spot a preset quantity of probes in each address in a reproducible and monitorable manner.

The bond of the probes on the solid support can be obtained both through covalent bonds and through high-affinity non-covalent bonds.

Covalent bonds can be obtained in different ways: by chemically modifying the oligonucleotides (for example, by adding amine, thiolic, mercapto or halogen groups), as disclosed by Guo et al. (1994) Nucleic Acid Res. 22: 5456-5465; by using substances with heterobifunctional reactive groups (such as 1-ethyl=3-(3-dimethylaminopropyl)-carbodiimide hydrochloride or EDAC), as disclosed by O'Donnel et al., (1991) Anal. Biochem 198: 138-142*;* Chrisey et al. (1996) Nucleic Acid Res.24: 3031-3039*;* Joos et al (1997) Anal. Biochem. 247: 96-101*;* with or without "post-spotting" treatment through 254 nm U.V. light or exposure to heat.

The halogen, amine, thiolic or mercapto group is added to the oligonucleotides in position 5'. Inserting a poly-T (polythymidine) tail that is between 5 and 20 mer in length in the oligonucleotides, still in position 5', is also provided for.

High-affinity non-covalent bonds can be obtained, for example, using biotinylated probes on supports modified by means of streptavidin.

The surfaces of the supports can, in turn, be chemically modified in different ways to promote the bond of the oligonucleotides (or of other macromolecules) by adding halogens, amine, or mercapto, functional groups as disclosed, for example, in: Lamture et al. (1994) Oligonucleotides Research 22: 2121-2125*;* Krieg et al. (1995) Nature 374: 546-549*;* Beattie et al. (1995) Mol. Biotechnol. 4: 213-225*;* Joos et al. (1997) Anal. Biochem 247: 96-101*;* Rogers et al. (1999) Anal. Biochem. 266: 23-30*;* Weiner et al. (1997) Proc. Natl. Acad. Sci. USA 94: 10833-10837*;* Boggs et al. (1997) Antisense Nucleic Acid Drug Dev. 7(5): 461-471*.*

The method provided by the invention above all requires extracting the genomic nucleic acids (DNA or RNA) from a sample to be analysed and/or from an enrichment broth, of the known type, in which the latter has been previously incubated. The sample may consist of water or of a foodstuff that can be contaminated, such as meat, fish, milk, sweet foods or may be a biological sample of human origin (pharyngeal swabs, bronchial aspirates, urogenital swabs, blood, biopsies, faeces).

Subsequently, simultaneous amplification of the various genic sequences present in the sample is provided for, which amplification is performed by means of multiplex PCR technique (which provides for the use of a set of primers that are specific for each of the target sequences searched for in the sample), if the genome of the pathogenic agent searched for is constituted by DNA or by means of multiplex RTPCR (Reverse Transcriptase PCR) technique, if the genome of the pathogenic agent searched for consists of RNA. The Multiplex PCR technique. The amplification procedure provides for the use of a pair of primers that amplify consensus regions for each of the target sequences searched for, that is for each pathogenic agent searched for. Each primer is an oligonucleotide that is able to trigger an amplification reaction catalysed by polymerase, at the end of which an amplified DNA is obtained. In order to be able to identify the amplified product obtained by PCR, marked primers are used, i.e. primers that have been modified in various ways. This can for example be obtained by inserting a biotin molecule, a digoxigenin molecule or a fluorochrome molecule on the end 5' of one or both the PCR primers.

Alternatively, the amplified product can be marked during PCR by incorporation of a nucleotide bound with a digoxigenin molecule or with another molecule that does not cause steric hindrance during the PCR reaction and subsequent hybridisation.

Once the amplified product has been obtained, the latter is denatured by heat treatment or by incubation in an alkaline environment. The denatured amplified product is then mixed with an appropriate hybridisation buffer and is essayed with the chip. Various hybridisation buffer (Maniatis) formulations exist, that basically vary from one another in their saline composition and in the type of "blocking" agent used to saturate the aspecific binding sites on the chip. Furthermore, "space-filling" macromolecules such as dextran sulphate are sometimes used to accelerate hybridisation kinetics.

Once the amplified product has been hybridised by the probe having a complementary sequence, this is highlighted by a technique that depends the manner in which the amplified product has been marked. If the latter has been marked with biotin or digoxigenin, the chip will be incubated with an antibody, conjugated for example with alkaline phosphatase or peroxidase, which antibody is directed against biotin or digoxigenin. Successive incubation of the chip with a specific substrate produces a colorimetric reaction in the exact site in which the enzyme and therefore the amplified hybridised product are present.

The analytical result can be read with the naked eye if chips 32 are used having an area of >1 cm². If on the other hand chips 32' are used having an area of <1 cm², an optic reader or scanner has to be used. The optic means vary according to the nature of the signal generated by the tracing molecule with which the amplified product has been marked. Tracing molecules that emit fluorescence require the use of sophisticated and expensive laser readers whereas the tracing molecules that develop a colorimetric reaction can be read by an optic reader using white light. In particular, an optic reader is provided (BCS Chip Reader) that enables the method for interpreting an analytical result provided by the invention and disclosed below to be applied.

Some examples are set out below in which the analytical system provided by the invention is applied to the search for bacteria and viruses in food, water and biological samples.

### Example 1

The method according to the invention has been used, in the form of an analytical kit, for searching and typing human Papillomavirus (HPV) in biological samples, comprising samples of exfoliated cervical cells and bioptic material fixed by formalin. Cancer of cervix uteri is still today the second cause of death due to tumours in the female sex. Epidemiological data clearly show that the occurrence of this tumour is closely linked to infections with some types of human Papillomavirus; the genome of this virus is found in nearly all cases of invasive cancer of the cervix. More than a hundred different types of HPV exist in nature, in addition to certain subtypes, and about thirty of these types are defined as anogenital because of their tendency to infect the mucose of the anogenital tract. Infection with genital HPVs is extremely frequent in the young sexually active population, but it fortunately clears up spontaneously and without leaving traces in the vast majority of cases. However, in a small percentage of cases, some genital HPVs, which are defined as "high oncogenic risk" HPVs, may establish persistent infections, causing neoplastic alterations to the mucous that may result in cancer. Nevertheless, such alterations may also persist for a long time without evolving and may even regress. The types of HPV considered to be high-risk are mainly represented by types 16, 18 and 45, which are the types most frequently found in invasive carcinomas. Other HPVs considered to be high-risk but which are less frequent than the former are types 26, 30, 31, 33, 35, 39, 51, 52, 53, 56, 58, 59, 66, 67, 68, 69, 70, 73 and 82. Genital HPVs are also known that never or only rarely cause invasive cancer. These are the low-risk HPVs, among which the types 6, 11, 34, 40, 42, 43, 44, and 54 are included. Some of these low-risk HPVs cause benign neoplasias such as warts and condylomas.

Cervical cancer evolves from dysplastic preinvasive lesions known as SIL (squamous intraepithelial lesions) or CIN (cervical intraepithelial lesions), which are classified in increasing degrees according to the seriousness of the alterations that are observed by microscopic analysis thereof.

These lesions are usually discovered and possibly removed by surgery during normal gynaecological examinations.

Nevertheless, since only a small number of these lesions is able to evolve, as already said, the aforementioned surgery is superfluous in most cases. The PAP test and colposcopy are essential for the diagnosis of these lesions, but unfortunately they cannot provide any type of information on their evolutive power.

However, it is known that the evolutive destiny of a given lesion depends on the type of HPV involved in the infection and on the duration of the infection. HPV typing is normally carried out by direct sequencing through hybridisation with type-specific molecular probes directed against the L1 viral region. A limit to this approach is constituted by the fact that the L1 region may be deleted during the process of integration of the viral DNA into the genome of the host cell, an essential stage in the viral transformation mechanism. If deletion of the L1 region occurs, the search for HPV and the corresponding typing provide an erroneously negative result, although the oncogenic power of the virus remains intact as it depends on the integrity of the E6 and E7 regions.

By applying the method provided by the invention, it is possible to significantly improve laboratory diagnostic procedures inasmuch as the HPV search and typing do not depend exclusively on the amplification of the L1 region. This result is obtained by using multiplex PCR, which enables the L1 and E6/E7 regions of high- and low-risk HPVs to be simultaneously amplified, followed by hybridisation with specific probes contained, for example, in the chips 32' of the microplate 30.

Many primers have been tested to amplify the L1 region of the HPV. The set of GPF/GP6+ primers was chosen because it has a wide spectrum, i.e. it is able to identify a wide range of HPVs, producing an amplified product of approximately 190 pairs of bases. This constitutes an excellent compromise between the need to increase the efficiency of the PCR (which is inversely proportionate to the length of the product) and the need to have a sufficiently long product for unequivocal typing of the latter. The sequences of the primers used for the amplification of the E6/E7 region of the low- and high-risk HPVs are disclosed in MO2000A000091 and in W00185994.

In the reaction mixture there are also present the b-GLOBL2 and b-GLOBR2 primers that are specific for human β-globin. The presence of these primers enables the quality of the nucleic acids preparation to be monitored, which preparation undergoes PCR. In fact, in the presence of an unsuitable preparation, no signal will be obtained for the β-globin. Furthermore, the presence of inhibitors of the PCR reaction is monitored owing to the presence of an Internal Control (IC) that is amplified by the β-globin primers. The two PCR products are nevertheless distinguishable through hybridisation, as the internal control possesses nucleotidic sequences that are not present in the β-globin. All the primers have been synthesised in such a way as to be provided with a tail of 11 Thymidine residues at the end 5', with which a digoxigenin molecule is bound. Tables 1-3 below respectively show the sequences of the primers and of the probes for searching for and typing HPV and the sequences amplified by the b-GLOBL2 and b-GLOBR2 primers.

**Table 1 (primers)**

| | |
|---|---|
| GPF | digoxigenin-5'-CAG-GGA-CAI-AAI-AAT-GGY-ATW-TG-3' |
| GP6+ | digoxigenin -5'-GAA-AAA-TAA-ACT-GTA-AAT-CAT-ATT-3' |
| pu-2R | digoxigenin -5'-GAG-CTG-TCG-CTT-AAT-TGC-TC 3' |
| ELRF | digoxigenin -5'IWA-CIG-TGG-AAG-AAG-ARA-C-3' |
| EHRF | digoxigenin -5'-AGA-YRT-TAT-AIT-TAT-TCI-GTR-TAT-GG -3' |
| b-GLOBL2 | digoxigenin -5'-CTT-TCA-GGG-CAA-TAA-TGA-3' |
| b-GLOBR2 | digoxigenin -5'-TGG-TAG-CTG-GAT-TGT-AGC-3' |

The GP primers identify a zone of about 190 pb on HPV region L1. The probes indicated below are comprised within this region. The pu-1M, -31B, 2R primers amplify consensus regions in the E6/E7 region of high-risk HPV types (pu2R-pu31B) and in the E6/E7 region of low-risk HPV types (pu2R-pu1M), forming amplified products with a molecular weight between 270-300 pb. The b-GLOBL2 and b-GLOBR2 primers amplify a segment of 178 pairs of bases of the gene of the β-globin (access number NG000007).

**Table 2 (probes)**

| Target | Specificity | Oligonucleotide 5' → 3' sequence | mer |
|---|---|---|---|
| 1. | HPV6 | ATCCGTAACTACATCTTCCACATACACCAA | 30 |
| 2. | HPV11 | ATCTGTGTCTAAATCTGCTACATACACTAA | 30 |
| 3. | HPV16 | GTCATTATGTGCTGCCATATCTACTTCAGA | 30 |
| 4. | HPV18 | TGCTTCTACACAGTCTCCTGTACCTGGGCA | 30 |
| 5. | HPV26 | TAGTACATTATCTGCAGCATCTGCATCC | 28 |
| 6. | HPV30 | TATCTGCAACCACACAAACGTTATCCAC | 28 |
| 7. | HPV31 | TGTTTGTGCTGCAATTGCAAACAGTGATAC | 30 |
| 8. | HPV33 | TTTATGCACACAAGTAACTAGTGACAGTAC | 30 |
| 9. | HPV34 | TACACAATCCACAAGTACAAATGCACCATA | 30 |
| 10. | HPV35 | GTCTGTGTGTTCTGCTGTGTCTTCTAGTGA | 30 |
| 11. | HPV39 | TCTACCTCTATAGAGTCTTCCATACCTTCT | 30 |
| 12. | HPV40 | GCTGCCACACAGTCCCCCACACCAACCCCA | 30 |
| 13. | HPV42 | CTGCAACATCTGGTGATACATATACAGCTG | 30 |
| 14. | HPV43 | TCTACTGACCCTACTGTGCCCAGTACATAT | 30 |
| 15. | HPV44 | GCCACTACACAGTCCCCTCCGTCTACATAT | 30 |
| 16. | HPV45 | ACACAAAATCCTGTGCCAAGTACATATGAC | 30 |
| 17. | HPV51 | AGCACTGCCACTGCTGCGGTTTCCCCAACA | 30 |
| 18. | HPV52 | TGCTGAGGTTAAAAAGGAAAGCACATATAA | 30 |
| 19. | HPV53 | ACTCTTTCCGCAACCACACAGTCTATGTCT | 30 |
| 20. | HPV54 | TACAGCATCCACGCAGGATAGCTTTAATAA | 30 |
| 21. | HPV56 | GTACTGCTACAGAACAGTTAAGTAAATATG | 30 |
| 22. | HPV58 | ATTATGCACTGAAGTAACTAAGGAAGGTAC | 30 |
| 23. | HPV59 | TCTGTGTGTGCTTCTACTACTTCTTCTAT | 29 |
| 24. | HPV66 | ACTATTAATGCAGCTAAAAGCACATTAACT | 30 |
| 25. | HPV67 | ACTTTATGTTCTGAGGAAAAATCAGAGGCT | 30 |
| 26. | HPV68 | TTTGTCTACTACTACTGAATCAGCTGT | 27 |
| 27. | HPV69 | AGTACTGTATCTGCACAATCTGCATCTGCC | 30 |
| 28. | HPV70 | ATTGTCTGCCTGCACCGAAACGGCCAT | 27 |
| 29. | HPV73 | AGGCTAGTAGCTCTACTACAACGTATGC | 28 |
| 30. | HPV82 | GTTTACTCCATCTGTTGCACAAACATTTAC | 30 |
| 31. | β-globin | CAATGTATCATGCCTCTTTGCACCATTCTA | 30 |
| 32. | Internal control | GGGTTACGTAAGCTACCTAGCTACTGCATG | 30 |
| 33. | PAN HPV L1 | TTTGTWACTGTIGTRGAYACIACMCGIAGTAC | 33 |
| 34. | HPVHR E6/E7 | CGTACAGAAACCCAGGTGTAATCATGCRTG CAGTATAATCATGCAYGGTAAAGTACCAAC GTGTAATAAMGCCATGCGTGGTAATGTACCACA ACGIAGIGAAACACAIGTIATTTTTTATGCATGGACC | 30 |
| | | | 30 |
| | | | 33 |
| | | | 39 |
| 35. | PAN HPV E6/E7 | TGYCAIAARCCITTRTGIMIAGIRGAAAA | 29 |

**Table 3 (amplified sequences of β-globin gene)**

| | | |
|---|---|---|
| Target | Name of the Primer | Sequence 5'-3' |
| B-globin | b-GLOBL2 | ctttcagggcaataatgatacaatgtatcatgcctctttgc accattctaaagaataacagtgataatttctgggttaaggc aatagcaatatctctgcatataaatatttctgcatataaat tgtaactgatgtaagaggtttcatattgctaatagcagcta caatccagctacca |
| Reference | b-GLOBR2 | |
| sequence | | |
| NCBI | | |
| NG000007 | | |
| Internal | b-GLOBL2 | ctttcagggcaataatgataggtacgtaagctacctagcta ctgcatgaataacagtgataatttctgggttaaggcaatag caatatctctgcatataaatatttctgcatataaattgtaa ctgatgtaagaggtttcatattgctaatagcagctacaatc cagctacca |
| Control | b-GLOBR2 | |
| | | |
| | | |
| | | |

The composition and the analytical procedure which may be used (BCS HPV KIT) in HPV search and typing with the HPV probes disclosed, are enlisted below.

### Composition of the kit

The kit comprises a Part A and a Part B. Part A comprises the reagents for the genic amplification, which reagents have to be stored at a temperature of < -20°C. Part B comprises the reagents for hybridisation and colorimetric detection on chip, which have to be stored at 2-8°C. In tables 4 and 5, set out below, the compositions of respectively Part A and Part B (for a 24-test kit) are shown in detail.

**Table 4 (Part A)**

| **N°** | **solution** | **Solution** | **Volume** | **Quantity** |
|---|---|---|---|---|
| 1 | **FM** | PCR premix for amplifying the L1 and E6/E7 regions of a wide range of HPV types, an endogenous internal control DNA and a synthetic internal control DNA; it consists of primers modified in 5' by digoxigenin, deoxyribonucleotides (dNTPs), PCR buffer, MgCl₂ and water. | | |
| | Consisting of: | | | |
| | -DNTP 2.5 mM 1% | | | |
| | -10xPCR Buffer 12.67% | | | |
| | -MgCl2(25mM) 12.67% | | | |
| | -Oligonucleotides 4% : | | | |
| | primer GPF (100 µM) | | | |
| | primer GP6+ (100 µM) | | | |
| | primer pu-2R (100 µM) | | | |
| | primer ELRF (100 µM) | | | |
| | primer EHRF (100 µM) | | 1.4 mL | 1 vial |
| | primer b-GLOBL2 (100 µM) | | | |
| | primer b-GLOBR2 (100 µM) | | | |
| 2 | **DNA Polymerase** 100% | DNA Polymerase | | |
| | | Enzyme(5U/µL) | 24 µL | 1 vial |
| 3 | **IC,** consisting of DNA IC 7% in water | PCR internal control | 100 µL | 1 vial |
| 4 | **CP,** consisting of HPV 42 DNA 7% in water | PCR positive control | 100 µL | 1 vial |

**Table 5 (part B)**

| | | | | |
|---|---|---|---|---|
| **N°** | **Solution** | **Description** | **Volume** | **Quantity** |
| 1 | BCS Hyb Buffer Consisting of: Maleic acid 1x 11.73 % NaCl 2.5 M 27.72% Boric acid 500 mM 1.56% NaOH 1N 6.27% Dextr. Sulf. Sodium 50.58% Herring Sperm DNA(10 g/bt) Ficoll TM PM400 1.01% PVP 1.01% Anti-VP1(100 µM) 0.1% | Hybridizating buffer that is formulated so as to promote the hybridization of the amplified products with the specific probes | 20 mL | 1 bottle |
| | Wash Buffer Consisting of: NaH2PO₄.2H₂O 1M 0.15% Na₂HPO₄.2H₂O 1M 0.54% NaCl 2.5 M 0.78% MgCl₂.1M 0.18% Urea 1% 7.69% | Washing buffer for removing the amplified products that are not bound after the hybridization phase | 80 mL | 1 bottle |
| 3 | Anti-Digoxigenin Antibody conjugated with Alkaline phosphatase (Roche Molecular Diagnostics) | The antibody binds specifically the digoxigenin of the amplified products hybridizated with the probes. The conjugated alkaline phosphatase (AP) is the enzyme that, in the presence of NBT/BCIP substrate, enables a colorimetric signal to be developed on the chip. | 24 µL | 1 vial |
| 4 | Blocking Reagent Consisting of: Maleic acid 1.2% NaCl 0.8% Solid NaOH 0.78% Blocking reagent(powder) 1% | Buffer that is formulated so as to reduce the non-specific bonds with the surface of the chip and to dilute the anti-Digoxigenin antibody solution. | 20 mL | 1 bottle |
| 5 | Detection Buffer Consisting of: HCL (12N) 0.1% Tris-base 1.2% NaCl 0.58% MgCl₂ 1% | Diluting buffer for the NBT/BCIP chromogenic substrate; it is formulated so as to increase the AP activity. | 40 mL | 1 bottle |
| 6 | NBT/BCIP 100% | Chromogenic substrate. The hydrolysis of BCIP (5-Bromo-4-chloro-3-indolyl phosphate indolyl phosphate), followed by the oxidation of NBT (Nitro blue tetrazolium) in the presence of AP, generates a blue precipitate on the localized zone of enzymatic activity. | 400 µL | 1 vial |
| 9 | BCS HPV Chip | Solid support for the hybridization reaction. Specific probes for each viral genotype, which is the target of the test, adhere to the surface of the support. | 24 Chip | 8 three chip - strips |

### Analytical procedure

### I. DNA Amplification

1. *Preparation of PCR Master Mix*. The reagents, which are shown in Table 6 below, are transferred to a PVC tube and mixed. For each analysis, an additional tube needs to be prepared for the PCR negative and positive control.

**Table 6**

| Reagents | Volume/tube | Number of tubes | Total volume |
|---|---|---|---|
| FM | 39.5 µL | × (n* + 1) | = ...µL |
| DNA Pol. | 0.5 µL | × (n* + 1) | = ...µL |
| IC | 0.5 µL | × (n* + 1) | = ...µL |

| | | | |
|---|---|---|---|
| *n = number of samples per test (including negative and positive controls) | | | |

2. 40 µL of master mix are delivered by means of a pipette into each PCR tube.
3. 10 µL of the extracted DNA are transferred to each tube and are mixed thoroughly; 10 µL of sterile water are added for the negative control and 10 µL of PC (DNA positive control) are added for the positive control. The final volume in each tube is 50 µL.
4. The PCR tubes are located in a thermocycler with heated lid and the following thermal profile is set: 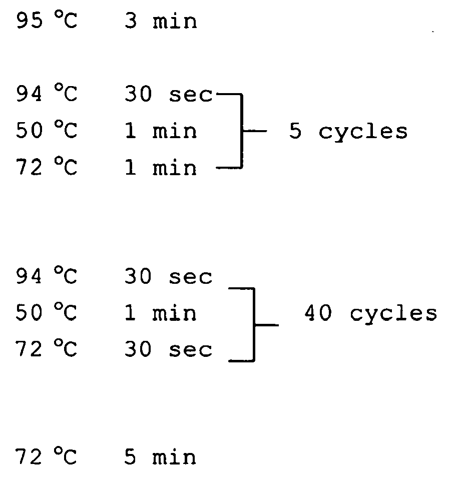
5. At the end of PCR, the hybridization phase is carried out or the amplified products are stored at -20 °C until the use thereof.

### II. Hybridization

### Preparation

1. The BCS Hyb Buffer and the Wash Buffer are taken to ambient temperature.
2. A suitable number of chips (as many as the samples to be tested, plus one for the negative control and one for the positive control) is taken to the same ambient temperature).

### Hybridization reaction

3. 20 µL of the amplified product are delivered by means of a pipette in 180 µL of BCS Hyb Buffer, in a 0.5-ml eppendorff-type tube, and are mixed thoroughly.
4. Denature at 99°C for 5 minutes.
5. Place in ice for another 5 minutes.
6. Each mix is transferred onto Transfer the mix onto a respective chip (BCS HPV Chip); the chips are incubated at 50 °C in a stove for 1 h, being slightly stirred if possible.

### IV. Stringency washing

1. Once the hybridization phase is completed, the liquid is removed from the chip.
2. 250 µL of Wash Buffer are added to the chip, the liquid is well distributed and then it is removed; this washing is repeated a second time.

### V. Blocking

1. A suitable quantity of the antibody anti-Digoxigenin is diluted with the Blocking Reagent, in a 1:2000 ratio (for example: 0.5 µl of antibody in 1 ml of Blocking reagent); 200 µL of this solution are transferred to each chip, which are incubated at ambient temperature, slightly stirred, for 30 minutes. The Blocking Reagent is then removed. 250 µL of Wash Buffer are added to the chip and two washes are carried out.
2. After the second wash, the residual liquid is poured out by upturning each chip on absorbent paper.

### VI. Colorimetric assay

1. 4 µL of NBT/BCIP are mixed with 196 µL of Detection Buffer, and the mixture is transferred onto each chip.
2. The reaction is then continued for 10 minutes in the dark and at ambient temperature.
3. The liquid is then removed, each chip is washed with 250 µL of water and the results are interpreted.

### VII. Interpretation of the results

The results originate from the interpretation of a specific pattern that forms on the chip through the effect of the hybridization reaction and the subsequent colorimetric reaction and owing to the preset arrangement of the probes on the chip. The probe-target hybrids are highlighted by the dark precipitate that is the result of the enzymatic reaction. The chip pattern can be read and interpreted by the naked eye for the > 1 cm² chips, such as those constituting the poly-chip in Figure 1, whilst it is necessary to use an optic reader (BCS Chip Reader) for the analysis of the < 1 cm² chips, such as those comprised in the microplates 30.

The test has been conducted correctly if the zones corresponding to the Hybridization Positive Control and to the PCR Positive Internal Control are evident. If one or more control zones are not visibile on the chip, the test must be repeated.

Figure 2 shows schematically, through symbols, the position of the probes fixed to the surface of the chip that is used in the analytical kit of example 1. In Table 7 below, the meanings of the symbols in Figure 2 are shown.

**Table 7**

| | | | |
|---|---|---|---|
| ci | Hybridization Control | 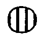 | HPV 16 |
| cp | PCR Internal Control | 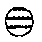 | HPV 18 |
| bg | β-globin | 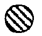 | HPV 26 |
| TA | High-risk types: region E6/E7 | 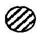 | HPV 30 |
| P | PAN HPV: region E6/E7 | 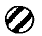 | HPV 31 |
| L | PAN HPV: region L1 | 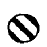 | HPV 33 |
| | | 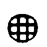 | HPV 35 |
| | | 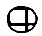 | HPV 39 |
| | | 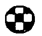 | HPV 45 |
| | | 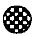 | HPV 51 |
| | | 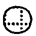 | HPV 52 |
| | | 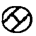 | HPV 53 |
| | | 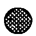 | HPV 56 |
| | | 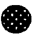 | HPV 58 |
| | | 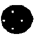 | HPV 59 |
| | | 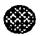 | HPV 66 |
| | | 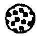 | HPV 67 |
| | | 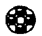 | HPV 68 |
| | | 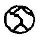 | HPV 69 |
| | | 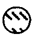 | HPV 70 |
| | | 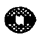 | HPV 73 |
| | | 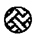 | HPV 82 |

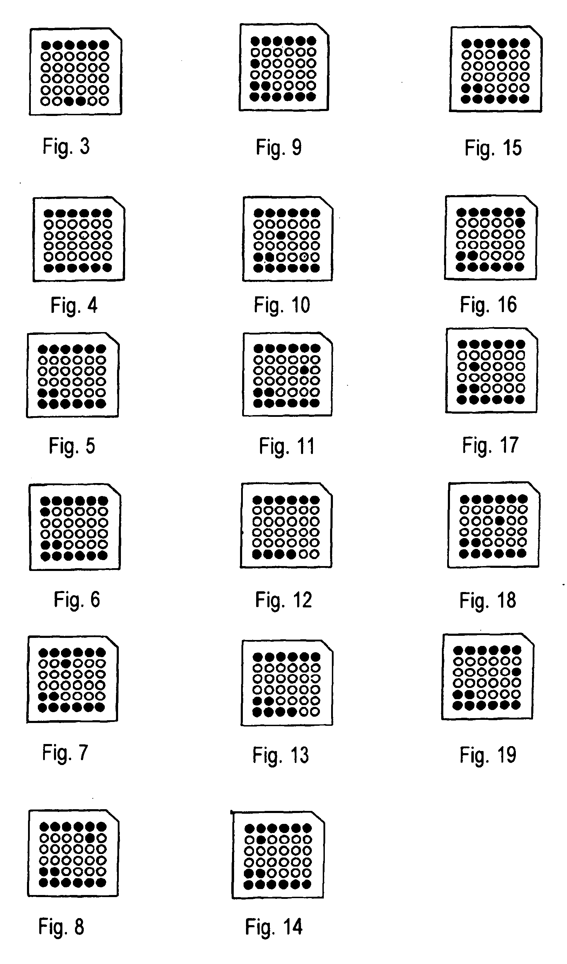

With reference to Figures 3 to 32, examples are shown of patterns and therefore of analytical results obtainable by the chip in Figure 2, depending on the presence or absence in an analysed sample of the various HPV types that are identifiable by the kit.

Figure 3 shows a negative sample.

Figure 4 shows a positive sample for low-risk HPV types.

Figure 5 shows a positive sample for high-risk HPV types, not comprised amongst the typable ones.

Figure 6 shows a positive sample for HPV 16.

Figure 7 shows a positive sample for HPV 26.

Figure 8 shows a positive sample for HPV 31.

Figure 9 shows a positive sample for HPV 35.

Figure 10 shows a positive sample for HPV 45.

Figure 11 shows a positive sample for HPV 52.

Figure 12 shows a positive sample for low-risk HPV types, with deletion in L1.

Figure 13 shows a positive sample for high-risk HPV types, not comprised amongst the typable ones, with deletion in L1.

Figure 14 shows a positive sample for HPV 18.

Figure 15 shows a positive sample for HPV 30.

Figure 16 shows a positive sample for HPV 33.

Figure 17 shows a positive sample for HPV 39.

Figure 18 shows a positive sample for HPV 51.

Figure 19 shows a positive sample for HPV 53.

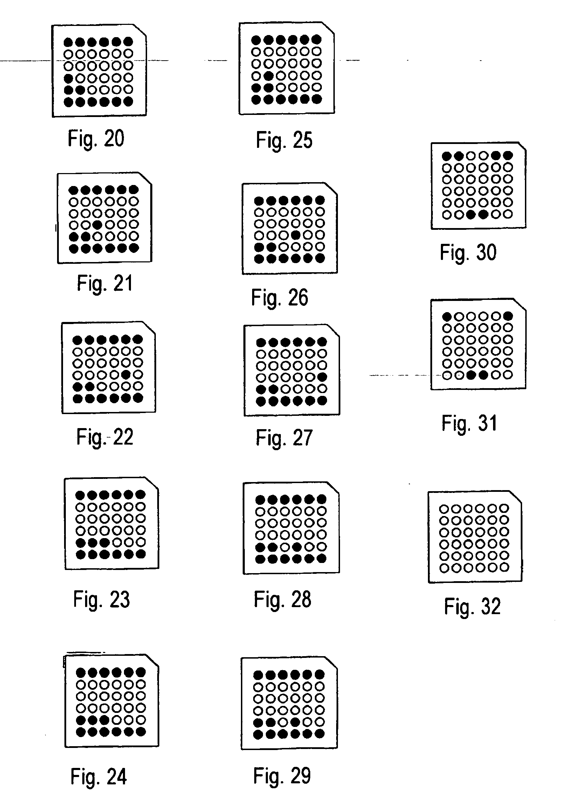

Figure 20 shows a positive sample for HPV 56.

Figure 21 shows a positive sample for HPV 59.

Figure 22 shows a positive sample for HPV 67.

Figure 23 shows a positive sample for HPV 69.

Figure 24 shows a positive sample for HPV 73.

Figure 25 shows a positive sample for HPV 58.

Figure 26 shows a positive sample for HPV 66.

Figure 27 shows a positive sample for HPV 68.

Figure 28 shows a positive sample for HPV 70.

Figure 29 shows a positive sample for HPV 82.

Figure 30 shows a sample in which the control for β-globin is absent, since the extraction procedure did not enable a suitable quantity of nucleic acid to be obtained.

Figure 31 shows a sample in which the PCR internal positive control is absent, since the extraction procedure did not enable a suitable nucleic acid to be obtained, for example due to the presence of possible inhibitors.

Figure 32 shows a sample in which hybridization internal controls are not present as some problem has affected the analysis also during the detection phase.

In the case of analytical results like those shown in Figures 30-32, the analysis has to be repeated.

To assess the performance of the above disclosed kit, the nucleic acid extracted from frozen vaginal spatula samples and frozen or paraffin-included biopsies has been parallely analysed using BCS HPV CHIP and ProDect HPV (the operation of which is described in the above-mentioned W00185994) as a reference test. The data emerging from this experimental comparison are set out in Table 8 below.

**Table 8**

| Regions | Number of samples (neg. + posit.) | Diagnostic specificity | Diagnostic sensitivity | Analytical sensitivity Copies/PCR |
|---|---|---|---|---|
| L1 | 30 | 96% | 98.5% | 10² |
| E6/E7 high-risk | 30 | 99.4 % | 97.4 % | 10³ |
| E6/E7 low-risk | 30 | 99.2 % | 96.3 % | 10³ |

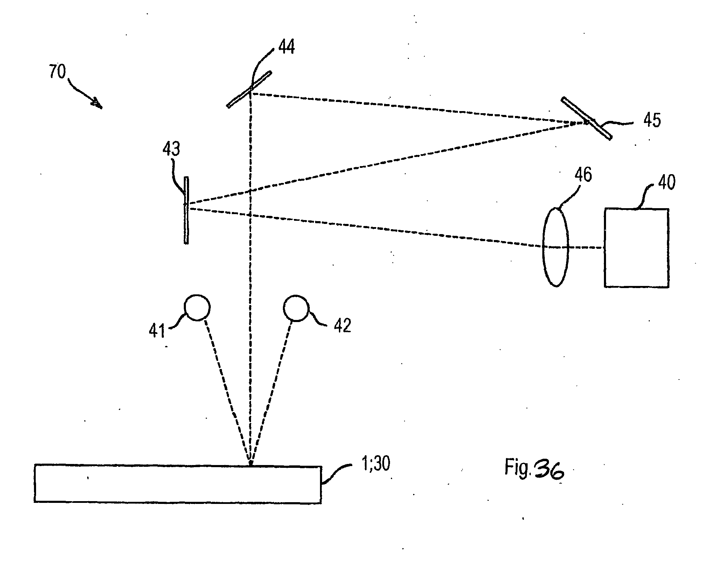

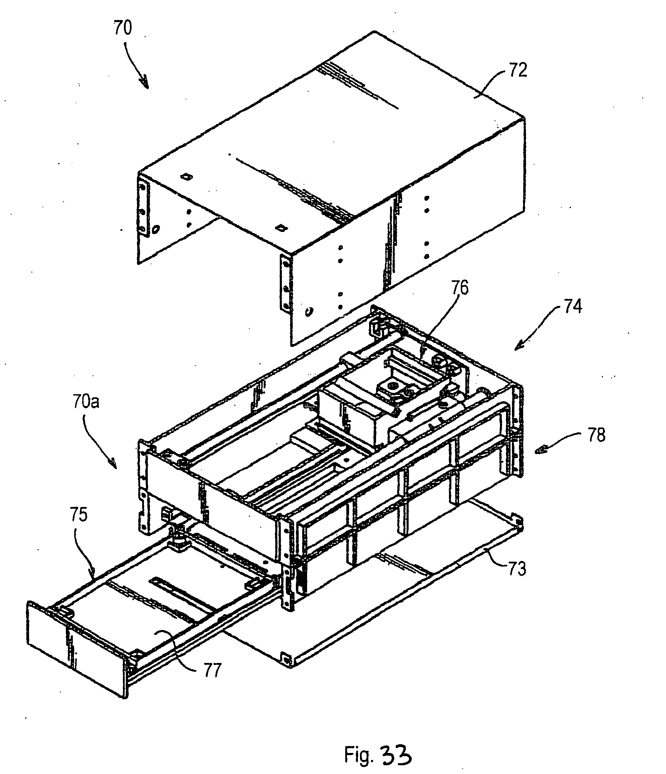

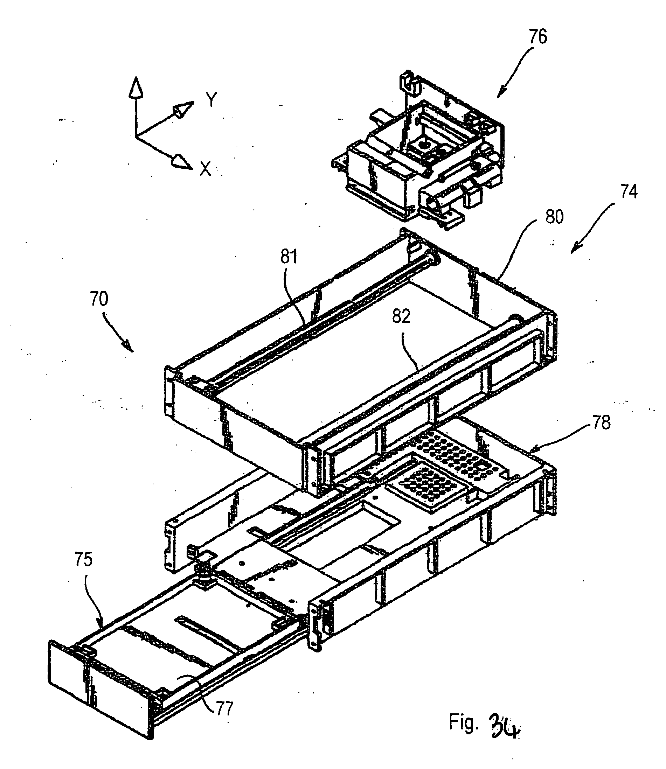

In order to enable reading of the chips 32' having a < 1 cm² area and/or to automate reading of the chips 32 having a > 1 cm² area, an apparatus is provided for acquiring an image from the chips 32 that are present in the container 1 (poly-chip) and/or from the chips 32' of the microplate 30. With reference to Figures 33-36, the apparatus comprises an optical unit 70, in which (Figure 33) a pair of light sources 41, 42 emitting white light, a plurality of mirrors 43-45, a focusing lens 46 and a sensor, for example a linear CCD 40, are present. The pair of light sources 41, 42 uniformly irradiate light inside the wells of the container 1 (poly-chip), or inside the wells 31 of the microplate 30, depending on the type of support used for the analysis. The lenses 43-45, suitably orientated inside the apparatus 70, retransmit the light reflected by the chips to the focusing lens 46 and then to the CCD 40. Since the aforementioned pair of light sources 41, 42 ensure uniform distribution of the light inside the wells, the contrast of the image and the intensity of the signal obtained from the reflection of the light are of good quality.

In addition to the optical unit 70, the apparatus comprises cooling means and a supply unit that are not shown.

With particular reference to Figures 33 to 35, the optical unit 70 comprises a base 73 and a lid 72, defining a space that is suitable for containing a scanning module 74. Onto a frontal portion 70a of the optical unit 70 an extractable drawer 75 is fitted in which a movable plate 77 is housed. In use, the microplate 30, or the poly-chip container 1 is located on the plate 77 when the latter is in an open position, i.e. extracted; thus by using a specific control an operator can automatically close the drawer 75 and start reading of the the chips 32. The scanning module 74 comprises an upper submodule 80 and a lower submodule 78.

In the upper submodule 80 an optic module 76 is housed, which can slide backwards and forwards, along a Y axis, owing to a pair of side guides 81, 82, fixed to opposite ends of the upper submodule 80.

The lower submodule 78 ensures suitable movement of the CCD of the optic module 76 along the Y axis and a suitable seat for the microplate 30 and/or the container 1. The lower submodule 78 comprises the extractable drawer 75, on a base portion 79 of which the movable plate 77 is fitted. The latter is movable along the Y axis, since it passively follows the movement of the drawer 75 when the latter is open and/or closed.

The optic module 76 comprises a control circuit (not shown) assembled on the rear part of the unit. The light sources 41, 42 are positioned in a lower portion, which is not shown, of the optic module. In use, the sources 41, 42, emit white light and irradiate the support that is to be read. A series of images of the chips 32, or 32', is generated by reflection of the light projected by the light sources 41, 42 through the mirrors 43-45. The image is then focused by the lens 46 and is received by the linear CCD 40.

With reference to Figure 37, CCD 40 starts scanning the wells (in the case shown, the wells 31 of a microplate 30) in direction A1→D1 along the Y axis. Subsequently, CCD 40 moves along a further X axis, orthogonal to the Y axis, and the wells in the direction A2→D2 are thus read, always along the Y axis. This sequence of operations is repeated until the wells are read in the direction A12→B12. The scan of the wells in the direction E12→H12 is thus performed, proceding with the wells in the direction E11→H11 and continuing until the wells in the direction E1→H are scanned.

The scan of the microplate is performed twice, and consequently the CCD 40 moves backwards and forwards for a sufficient time so that the entire microplate 30 can be read, that is for about three minutes. Owing to this double scanning, it is possible to reduce the dimension of the images to be analysed.

The images of the chips 32, 32' obtained by the apparatus disclosed above can be acquired and managed by a suitable computer programme. The programme can perform automatically a comparison between the image of a given pattern, which has been produced on the chip 32, 32' by means of hybridization highlighted by a colorimetric reaction, with a series of images of reference patterns (templates) that have been previously defined and stored. The programme is able to create image files in a sequential manner. Once the image of a pattern has been acquired, the position of the dark areas, or spots, forming each pattern is compared with the position of the spots forming the patterns of the reference images. The operator can create a database of images to be used during subsequent analyses.

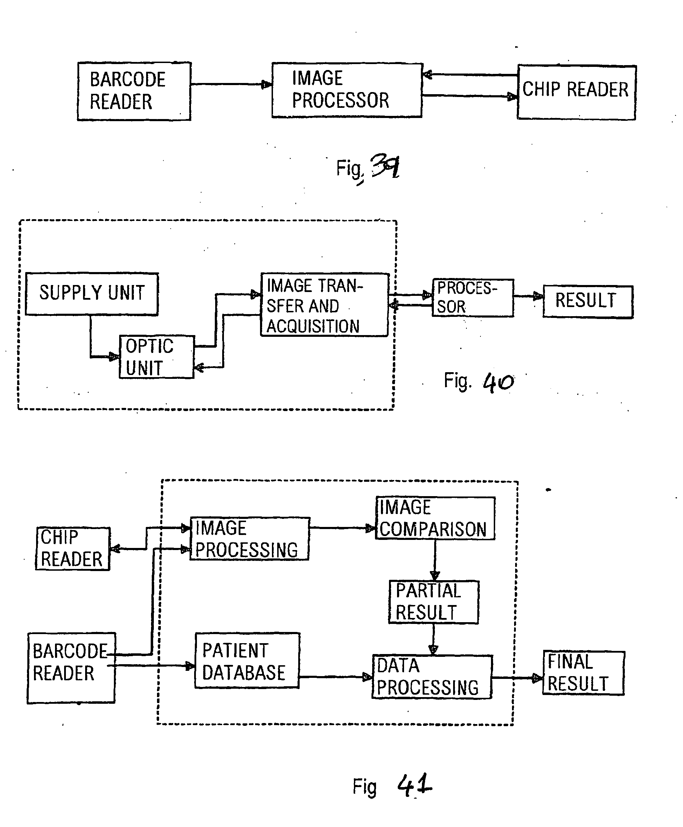

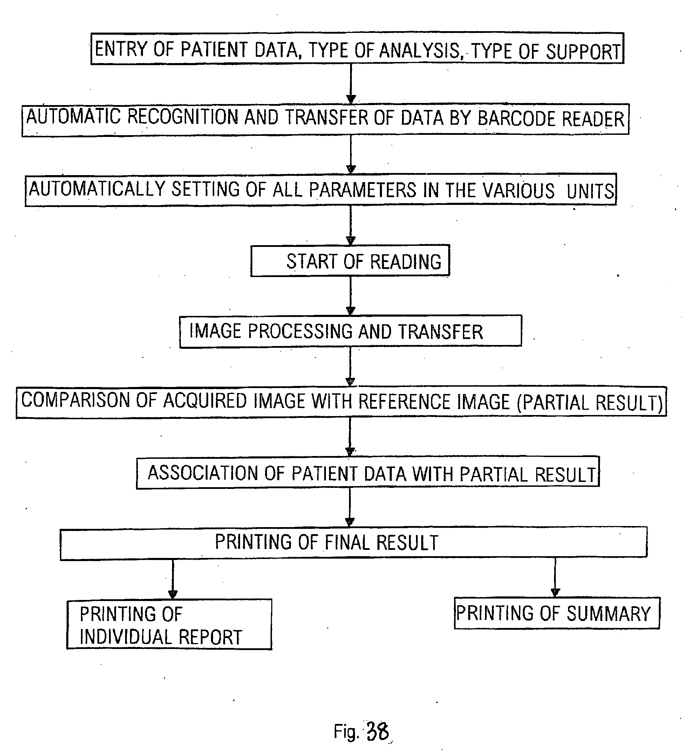

With reference to Figures 38 to 41, the possibility is provided of supplementing the reading apparatus disclosed above with further optic means, for example a bar code optic reader, of the known type. An arrangement (Figure 57) is obtained in this way comprising the bar code optic reader, the apparatus or chip reader disclosed above and an image processor, that is the computer on which the aforementioned programme for acquiring and managing images is run. In use, the three apparatuses reciprocally cooperate enabling the automatic management of data that are not directly analytical but nevertheless relate to the sample subjected to analysis to be associated with the interpretation of the images coming from the chips 32, 32'.

The operator, before starting to read the chips 32, 32', acquires information regarding the analysed sample, for example information on the origin of the sample and/or on the type of analysis made and therefore on the type of chip used, which information is contained in a bar code printed on an adhesive label. The latter can be applied, for example, to the paper documentation that normally accompanies each sample in a laboratory, or can be applied to the container of the sample.

The programme for acquiring and managing images that is used to check the operation of the chip reader is also able to check the operation of the bar code optic reader. In this way, the data that are acquired through the latter are sent to the computer and stored therein.

Then the operator, by acting on the computer, sets the suitable reading parameters of the chip reader and, after positioning the microplate 30 or the container 1 in the drawer 75, starts reading. The chip reader acquires the images coming from the chips 32, 32' and sends them to the computer, where the comparison between the acquired images of the patterns and the reference images is made. In this phase, the programme produces a so-called partial result, i.e. an analytical result, which is positive or negative depending on circumstances, which is not yet associated with the information on the sample that was previously acquired through the reading of the barcode. In a subsequent phase, the programme calls up from the computer memory the information on the sample and associates the information with the partial result, producing a final result in which the outcome of the analysis is associated with the information on the sample. This final result can be printed on paper in such a way as to produce an analytical report, and the programme can both print a single final result and a summarising printout, for example of the final results obtained during a certain period of laboratory activity.

Owing to the procedure disclosed above, the activity performed in a laboratory is significantly facilitated inasmuch as the time that is normally required for processing the analytical reports is substantially reduced.

## Claims

1. Miniaturized solid support for searching for and identifying a Human Papilloma Virus selected from a group comprising: HPV 6, HPV11, HPV16, HPV18, HPV26, HPV30, HPV31, HPV33, HPV34, HPV35, HPV39, HPV40, HPV42, HPV43, HPV44, HPV45, HPV51, HPV52, HPV53, HPV 54, HPV56, HPV58, HPV59, HPV66, HPV67, HPV68, HPV69, HPV70, HPV73, HPV82, **characterized in that** said solid support comprises at least the following oligonucleotidic probes with sequence:
5'-ATCCGTAACTACATCTTCCACATACACCAA-3' ,
5'-ATCTGTGTCTAAATCTGCTACATACACTAA-3' ,
5'-GTCATTATGTGCTGCCATATCTACTTCAGA-3',
5'-TGCTTCTACACAGTCTCCTGTACCTGGGCA-3' ,
5'-TAGTACATTATCTGCAGCATCTGCATCC-3,
5'-TATCTGCAACAACACAAACGTTATCCAC-3' ,
5'-TGTTTGTGCTGCAATTGCAAACAGTGATAC-3',
5'-TTTATGCACACAAGTAACTAGTGACAGTAC-3' ,
5'-TACACAATCCACAAGTACAAATGCACCATA-3' ,
5' -GTCTGTGTGTTCTGCTGTGCTTTCTAGTGA-3' ,
5'-TCTACCTCTATAGAGTCTTCCATACCTTCT-3' ,
5'-GCTGCCACACAGTCCCCCACACCAACCCCA-3' ,
5'-CTGCAACATCTGGTGATACATATACAGCTG-3',
5'-TCTACTGACCCTACTGTGCCCAGTACATAT-3',
5' -GCCACTACACAGTCCCCTCCGTCTACATAT-3',
5'-ACACAAAATCCTGTGCCAAGTACATATGAC-3',
5'-AGCACTGCCACTGCTGCGGTTTCCCCAACA-3',
5'-TGCTGAGGTTAAAAAGGAAAGCACATATAA-3' ,
5'-ACTCTTTCCGCAACCACACAGTCTATGTCT-3',
5'-TACAGCATCCACGCAGGATAGCTTTAATAA-3',
5'-GTACTGCTACAGAACAGTTAAGTAAATATG-3',
5'-ATTATGCACTGAAGTAACTAAGGAAGGTAC-3' ,
5'-TCTGTGTGTGCTTCTACTACTTCTTCTAT-3',
5'-ACTATTATTGCAGCTAAAAGCACATTAACT-3',
5'-ACTTTATGTTCTGAGGAAAAATCAGAGGCT-3' ,
5'-TTTGTCTACTACTACTGAATCAGCTGT-3' ,
5'-AGTACTGTATCTGCACAATCTGCATCTGCC-3',
5'-ATTGTCTGCCTGCACCGAAACGGCCAT-3',
5'-AGGCTAGTAGCTCTACTACAACGTATGC-3',
5'-GTTTACTCCATCTGTTGCACAAACATTTAC-3',
5'-CAATGTATCATGCCTCCTTGCACCATTCTA-3',
5'-TTTGTWACTGTIGTRGAYACIACMCGIAGTAC-3',
5'-CGTACAGAAACCCAGGTGTAATCATGCRTG-3',
5'-CAGTATAATCATGCAYGGTAAAGTACCAAC-3',
5'-GTGTAATAAMGCCATGCGTGGTAATGTACCACA-3',
5'-ACGIAGIGAAACACAIGTITATTTTTTTATGCATGGACC-3',
5'-TGYCAIAARCCITTRTGIMIAGIRGAAAA-3'.
wherein said oligonucleotidic probes adhere to preset sites of said solid support.

2. Miniaturized solid support according to claim 1, furthermore comprising an internal control having the following sequence:
5'-GGGTTACGTAAGCTACCTAGCTACTGCATG-3'.

3. Miniaturized solid support according to claims 1-2, said oligonucleotidic probes are provided, in 5' position, with a polythymidine tail.

4. Miniaturized solid support according to claims 1-3, wherein said oligonucleotidic probes are provided, in position 5', with a molecule selected from a group comprising: biotin, mercapto group, amino group, halogen group.

5. Miniaturized solid support according to any one of claims 1 -4 , having an area of > 1 cm²_{.}

6. Miniaturized solid support according to any one of claims 1 - 5, having an area of < 1 cm²_{.}

7. Analytical kit for searching for and identifying a Human Papilloma Virus in a sample, comprising a Miniaturized solid support according to claims 1-6, gene amplifying means for obtaining an amplification of specific genic sequences contained in said genomic nucleic acids, and means suitable for detection of said hybridization by optic means.

8. Kit according to claim 14, wherein said gene amplifying means comprises primers selected from the following sequences:
digoxigenin-5'-CAGGGACAIAAIAATGGYATWTG-3',
digoxigenin- 5'-GAAAAATAAACTGTAAATCATATT-3',
digoxigenin-5'-GAGCTGTCGCTTAATTGCTC-3',
digoxigenin-5'-IWACIGTGGAAGAAGARAC-3',
digoxigenin-5'-AGAYRTTATAITTATTCIGTRTATGG-3',
digoxigenin-5'-CTTTCAGGGCAATAATGA-3',
digoxigenin-5'-TGGTAGCTGGATTGTAGC-3'.

9. Kit according to claim 8, further comprising an amplification internal control.

10. Kit according to any one of claims 7-9 , furthermore comprising a synthetic oligonucleotidic sequence that acts as a control for the presence of inhibitors of a PCR reaction.

11. Kit according to any one of claims 7-10 , wherein said means suitable for detection comprises colorimetric means.

12. Kit according to claim 11, wherein said colorimetric means comprises a chromogenic substrate that is based on 5-bromo-4-chloro-indolyl-phosphate and nitro-blue-tetrazole.

13. Kit according to any one of claims 7-12, further comprising an anti-digoxigenin antibody, which is conjugated with alkaline phosphatase.

## Patentansprüche

1. Miniaturisierter fester Träger zum Suchen nach einem und zum Identifizieren eines humanen Papilloma-Virus ausgewählt aus der Gruppe umfassend HPV6, HPV11, HPV16, HPV18, HPV26, HPV30, HPV31, HPV33, HPV34, HPV35, HPV39, HPV40, HPV42, HPV43, HPV44, HPV45, HPV51, HPV52, HPV53, HPV54, HPV56, HPV58, HPV59, HPV66, HPV67, HPV68, HPV69, HPV70, HPV73, HPV82, **dadurch gekennzeichnet, dass** der feste Träger wenigstens eine der nachfolgenden Oligonukleotidsonden mit der Sequenz aufweist:
5'-ATCCGTAACTACATCTTCCACATACACCAA-3',
5'-ATCTGTGTCTAAATCTGCTACATACACTAA-3',
5'-GTCATTATGTGCTGCCATATCTACTTCAGA-3',
5'-TGCTTCTACACAGTCTCCTGTACCTGGGCA-3',
5'-TAGTACATTATCTGCAGCATCTGCATCC-3',
5'-TATCTGCAACAACACAAACGTTATCCAC-3',
5'-TGTTTGTGCTGCAATTGCAAACAGTGATAC-3',
5'-TTTATGCACACAAGTAACTAGTGACAGTAC-3',
5'-TACACAATCCACAAGTACAAATGCACCATA-3',
5'-GTCTGTGTGTTCTGCTGTGCTTTCTAGTGA-3',
5'-TCTACCTCTATAGAGTCTTCCATACCTTCT-3',
5'-GCTGCCACACAGTCCCCCACACCAACCCCA-3',
5'-CTGCAACATCTGGTGATACATATACAGCTG-3',
5'-TCTACTGACCCTACTGTGCCCAGTACATAT-3',
5'-GCCACTACACAGTCCCCTCCGTCTACATAT-3',
5'-ACACAAAATCCTGTGCCAAGTACATATGAC-3',
5'-AGCACTGCCACTGCTGCGGTTTCCCCAACA-3',
5'-TGCTGAGGTTAAAAAGGAAAGCACATATAA-3',
5'-ACTCTTTCCGCAACCACACAGTCTATGTCT-3',
5'-TACAGCATCCACGCAGGATAGCTTTAATAA-3',
5'-GTACTGCTACAGAACAGTTAAGTAAATATG-3',
5'-ATTATGCACTGAAGTAACTAAGGAAGGTAC-3',
5'-TCTGTGTGTGCTTCTACTACTTCTTCTAT-3',
5'-ACTATTATTGCAGCTAAAAGCACATTAACT-3',
5'-ACTTTATGTTCTGAGGAAAAATCAGAGGCT-3',
5'-TTTGTCTACTACTACTGAATCAGCTGT-3',
5'-AGTACTGTATCTGCACAATCTGCATCTGCC-3',
5'-ATTGTCTGCCTGCACCGAAACGGCCAT-3',
5'-AGGCTAGTAGCTCTACTACAACGTATGC-3',
5'-GTTTACTCCATCTGTTGCACAAACATTTAC-3',
5'-CAATGTATCATGCCTCCTTGCACCATTCTA-3',
5'-TTTGTWACTGTIGTRGAYACIACMCGIAGTAC-3',
5'-CGTACAGAAACCCAGGTGTAATCATGCRTG-3',
5'-CAGTATAATCATGCAYGGTAAAGTACCAAC-3',
5'-GTGTAATAAMGCCATGCGTGGTAATGTACCACA-3',
5'-ACGIAGIGAAACACAIGTITATTTTTTTATGCATGGACC-3',
5'-TGYCAIAARCCITTRTGIMIAGIRGAAAA-3',
wobei die Oligonukleotidproben an vorgewählten Stellen des festen Trägers befestigt sind.

2. Miniaturisierter fester Träger nach Anspruch 1, des Weiteren umfassend eine interne Kontrolle mit der nachfolgenden Sequenz:
5'-GGGTTACGTAAGCTACCTAGCTACTGCATG-3'.

3. Miniaturisierter fester Träger nach Anspruch 1 oder 2, wobei die Oligonukleotidsonden an der 5'-Position mit einem Polythymidinschwanz versehen sind.

4. Miniaturisierter fester Träger nach einem der Ansprüche 1 bis 3, wobei die Oligonukleotidsonden an der 5'-Position mit einem Molekül ausgewählt aus der Gruppe umfassend Biotin, Mercaptogruppe, Aminogruppe, Halogengruppe versehen sind.

5. Miniaturisierter fester Träger nach einem der Ansprüche 1 bis 4 mit einer Fläche von mehr als 1 cm².

6. Miniaturisierter fester Träger nach einem der Ansprüche 1 bis 4 mit einer Fläche von weniger als 1 cm².

7. Analysen-Kit zum Suchen nach einem und zum Identifizieren eines humanen Pailloma-Virus in einer Probe umfassend einen miniaturisierten festen Träger nach einem der Ansprüche 1 bis 6, Gen amplifizierende Mittel zum Erhalten einer Amplifikation von spezifischen Gensequenzen, welche in den genomischen Nukleinsäuren enthalten sind, sowie Mittel, welche zur Detektion von Hybridisierung durch optische Mittel geeignet sind.

8. Kit nach Anspruch 7, wobei das Gen amplifizierende Mittel Primer enthält, welche aus den nachfolgenden Sequenzen ausgewählt sind:
Digoxigenin-5'-CAGGGACAIAAIAATGGYATWTG-3',
Digoxigenin-5'-GAAAAATAAACTGTAAATCATATT-3',
Digoxigenin-5'-GAGCTGTCGCTTAATTGCTC-3',
Digoxigenin-5'-IWACIGTGGAAGAAGARAC-3',
Digoxigenin-5'-AGAYRTTATAITTATTCIGTRTATGG-3',
Digoxigenin-5'-CTTTCAGGGCAATAATGA-3',
Digoxigenin-5'-TGGTAGCTGGATTGTAGC-3'.

9. Kit nach Anspruch 8, des Weiteren enthaltend eine interne Amplifikationskontrolle.

10. Kit nach einem der Ansprüche 7 bis 9, des Weiteren enthaltend eine synthetische Oligonukleotidsequenz, welche als eine Kontrolle für die Anwesenheit von Inhibitoren einer PCR-Reaktion agiert.

11. Kit nach einem der Ansprüche 7 bis 10, wobei die zur Detektion geeigneten Mittel kolorimetrische Mittel umfassen.

12. Kit nach Anspruch 11, wobei das kolorimetrische Mittel ein chromogenes Substrat enthält, welches auf 5-Brom-4-chlorindolyl-phosphat und Nitroblautetrazol basiert.

13. Kit nach einem der Ansprüche 7 bis 12, des Weiteren enthaltend einen Antidigoxigenin-Antikörper, welcher mit alkalischer Phosphatase konjugiert ist.

## Revendications

1. Support solide miniaturisé pour la recherche et l'identification d'un Virus de Papillome Humain sélectionné dans un groupe comprenant: HPV 6, HPV11, HPV16, HPV18, HPV26, HPV30, HPV31, HPV33, HPV34, HPV35, HPV39, HPV40, HPV42, HPV43, HPV44, HPV45, HPV51, HPV52, HPV53, HPV54, HPV56, HPV58, HPV59, HPV66, HPV67, HPV68, HPV69, HPV70, HPV73, HPV82, **caractérisé en ce que** ledit support solide comprend au moins les sondes oligonucléotidiques suivantes avec la séquence:
5'-ATCCGTAACTACATCTTCCACATACACCAA-3' ,
5'-ATCTGTGTCTAAATCTGCTACATACACTAA-3' ,
5'-GTCATTATGTGCTGCCATATCTACTTCAGA-3',
5'-TGCTTCTACACAGTCTCCTGTACCTGGGCA-3' ,
5'-TAGTACATTATCTGCAGCATCTGCATCC-3,
5' -TATCTGCAACAACACAAACGTTATCCAC-3',
5'-TGTTTGTGCTGCAATTGCAAACAGTGATAC-3',
5'-TTTATGCACACAAGTAACTAGTGACAGTAC-3',
5'-TACACAATCCACAAGTACAAATGCACCATA-3',
5'-GTCTGTGTGTTCTGCTGTGCTTTCTAGTGA-3',
5'-TCTACCTCTATAGAGTCTTCCATACCTTCT-3',
5'-GCTGCCACACAGTCCCCCACACCAACCCCA-3',
5'-CTGCAACATCTGGTGATACATATACAGCTG-3',
5'-TCTACTGACCCTACTGTGCCCAGTACATAT-3',
5'-GCCACTACACAGTCCCCTCCGTCTACATAT-3',
5'-ACACAAAATCCTGTGCCAAGTACATATGAC-3',
5'-AGCACTGCCACTGCTGCGGTTTCCCCAACA-3',
5'-TGCTGAGGTTAAAAAGGAAAGCACATATAA-3',
5'-ACTCTTTCCGCAACCACACAGTCTATGTCT-3',
5'-TACAGCATCCACGCAGGATAGCTTTAATAA-3',
5'-GTACTGCTACAGAACAGTTAAGTAAATATG-3',
5'-ATTATGCACTGAAGTAACTAAGGAAGGTAC-3',
5'-TCTGTGTGTGCTTCTACTACTTCTTCTAT-3',
5'-ACTATTATTGCAGCTAAAAGCACATTAACT-3',
5'-ACTTTATGTTCTGAGGAAAAATCAGAGGCT-3',
5'-TTTGTCTACTACTACTGAATCAGCTGT-3',
5'-AGTACTGTATCTGCACAATCTGCATCTGCC-3',
5'-ATTGTCTGCCTGCACCGAAACGGCCAT-3',
5'-AGGCTAGTAGCTCTACTACAACGTATGC-3',
5'-GTTTACTCCATCTGTTGCACAAACATTTAC-3',
5'-CAATGTATCATGCCTCCTTGCACCATTCTA-3',
5'-TTTGTWACTGTIGTRGAYACIACMCGIAGTAC-3',
5'-CGTACAGAAACCCAGGTGTAATCATGCRTG-3',
5'-CAGTATAATCATGCAYGGTAAAGTACCAAC-3',
5'-GTGTAATAAMGCCATGCGTGGTAATGTACCACA-3',
5'-ACGIAGIGAAACACAIGTITATTTTTTTATGCATGGACC-3',
5'-TGYCAIAARCCITTRTGIMIAGIRGAAAA-3'.
où lesdites sondes oligonucléotidiques adhèrent à des sites préétablis dudit support solide.

2. Support solide miniaturisé selon la revendication 1, comprenant de plus un contrôle interne ayant la séquence suivante:
5'-GGGTTACGTAAGCTACCTAGCTACTGCATG-3'.

3. Support solide miniaturisé selon les revendications 1-2, lesdites sondes oligonucléotidiques sont prévues à la position 5', pourvues d'une queue de polythymidine.

4. Support solide miniaturisé selon les revendications 1-3, où lesdites sondes oligonucléotidiques sont pourvues, à la position 5', d'une molécule sélectionnée dans un groupe comprenant: biotine, groupe mercapto, groupe amino, groupe halogène.

5. Support solide miniaturisé selon l'une quelconque des revendications 1-4 ayant une aire de > 1 cm².

6. Support solide miniaturisé selon l'une quelconque des revendications 1-5 ayant une aire de < 1 cm².

7. Kit analytique pour la recherche et l'identification d'un Virus de Papillome Humain dans un échantillon comprenant un support solide miniaturisé selon les revendications 1-6, un moyen d'amplification de gène pour obtenir une amplification de séquence génique spécifique contenue dans lesdits acides nucléiques génomiques et un moyen approprié pour la détection de ladite hybridation par un moyen optique.

8. Kit selon la revendication 14 où ledit moyen d'amplification de gène comprend des amorces sélectionnées parmi les séquences suivantes:
digoxigénine-5'-CAGGGACAIAAIAATGGYATWTG-3',
digoxigénine-5'-GAAAAATAAACTGTAAATCATATT-3',
digoxigénine-5'-GAGCTGTCGCTTAATTGCTC-3',
digoxigénine-5'-IWACIGTGGAAGAAGARAC-3',
digoxigénine-5'-AGAYRTTATAITTATTCIGTRTATGG-3',
digoxigénine-5'-CTTTCAGGGCAATAATGA-3',
digoxigénine-5'-TGGTAGCTGGATTGTAGC-3'.

9. Kit selon la revendication 8, comprenant de plus un contrôle interne d'amplification.

10. Kit selon l'une quelconque des revendications 7-9, comprenant de plus une séquence oligonucléotidique synthétique qui agit comme contrôle pour la présence d'inhibiteurs d'une réaction de PCR.

11. Kit selon l'une quelconque des revendications 7-10, où ledit moyen approprié pour la détection comprend un moyen colorimétrique.

12. Kit selon la revendication 11, où ledit moyen colorimétrique comprend un substrat chromogène qui est basé sur le 5-bromo-4-chloro-indolyl-phosphate et le bleu-nitro-tétrazole.

13. Kit selon l'une quelconque des revendications 7-12 comprenant de plus un anticorps anti-digoxigénine, qui est conjugué à la phosphatase alcaline.
